Europäisches Patentamt

European Patent Office

Office européen des brevets

(19)

(11) EP 1 218 329 B1

(12) FASCICULE DE BREVET EUROPEEN

(45) Date de publication et mention
de la délivrance du brevet:
**25.02.2004 Bulletin 2004/09**

(51) Int Cl.7: **C07C 51/353**, C07C 53/08,
C07C 51/12, C07C 67/36,
C07C 69/14

(21) Numéro de dépôt: **00951624.6**

(22) Date de dépôt: **21.06.2000**

(86) Numéro de dépôt international:
**PCT/FR2000/001706**

(87) Numéro de publication internationale:
**WO 2000/078700 (28.12.2000 Gazette 2000/52)**

(54) **PROCEDE POUR AMELIORER LA STABILITE ET/OU EVITER LA DESACTIVATION DU CATALYSEUR LORS DE LA FABRICATION D'ACIDE ACETIQUE ET/OU D'ACETATE DE METHYLE**

VERFAHREN ZUM VERBESSERN DER STABILITAET UND/ODER ZUM VERMEIDEN DER DESAKTIVIERUNG DES KATALYSATORS BEI DER HERSTELLUNG VON ESSIGSAEURE UND/ODER METHYLACETAT

METHOD FOR IMPROVING THE STABILITY AND/OR AVOIDING DEACTIVATION OF A CATALYST WHEN MAKING AN ACETIC ACID AND/OR METHYL ACETATE

(84) Etats contractants désignés:
**AT BE CH CY DE DK ES FI FR GB GR IE IT LI LU MC NL PT SE**

(30) Priorité: **22.06.1999 FR 9907916**

(43) Date de publication de la demande:
**03.07.2002 Bulletin 2002/27**

(73) Titulaire: **Acetex Chimie**
**92200 Neuilly-sur-Seine (FR)**

(72) Inventeurs:
• **THIEBAUT, Daniel**
  **F-64230 Lescar (FR)**

• **PATOIS, Carl**
  **F-68400 Riedisheim (FR)**
• **LAYEILLON, Lise**
  **F-64170 AUDEJOS (FR)**
• **MARCHAND, Daniel**
  **F-64110 Jurançon (FR)**

(74) Mandataire: **Giraud, Françoise et al**
**Cabinet Beau de Loménie**
**158, rue de l'Université**
**75340 Paris Cedex 07 (FR)**

(56) Documents cités:
**WO-A-97/35828**

EP 1 218 329 B1

**Description**

La présente invention a pour objet un procédé pour améliorer la stabilité et/ou éviter la désactivation du catalyseur dans les procédés de fabrication d'acide acétique et/ou d'acétate de méthyle ainsi qu'un procédé complet de fabrication d'acide acétique et/ou d'acétate de méthyle comprenant ce procédé.

Plus précisément, la présente invention a pour objet un procédé perfectionné permettant d'améliorer la stabilité et/ou d'éviter la désactivation du catalyseur dans le cas des procédés de préparation d'acide acétique et/ou d'acétate de méthyle par isomérisation du formiate de méthyle et éventuellement par carbonylation du méthanol, en présence d'un système catalytique comprenant au moins un promoteur halogéné et au moins un composé à base d'iridium.

L'invention concerne également des conditions particulières où ce procédé de stabilisation s'applique de façon particulièrement avantageuse.

Diverses voies d'accès à l'acide acétique et/ou à l'acétate de méthyle sont connues et exploitées industriellement. Parmi celles-ci figure la réaction de carbonylation du méthanol mise en oeuvre en phase liquide, sous pression de monoxyde de carbone qui est l'un des réactifs, en présence d'un système catalytique homogène. Une autre voie d'accès à l'acide acétique consiste à effectuer l'isomérisation du formiate de méthyle. Cette réaction est, elle-même, généralement réalisée en présence d'un système catalytique en phase homogène. Enfin, selon un autre procédé, on réalise simultanément la carbonylation du méthanol et l'isomérisation du formiate de méthyle.

Le procédé de carbonylation au rhodium est un procédé connu, exploité industriellement et ayant fait l'objet de nombreux articles et brevets comme, par exemple. les brevets américains US 3,769,329 et US 3,813,428.

Les brevets européens EP 618 183 et EP 618 184 ainsi que les brevets européens EP 785 919 et EP 759 022 décrivent un procédé de carbonylation en présence d'un système catalytique à base d'iridium et, le cas échéant, contenant en plus du rhodium.

Un procédé de carbonylation à l'iridium et au ruthénium, actuellement exploité industriellement, est décrit dans le brevet européen EP 643 034.

Plus récemment, une nouvelle voie d'accès constituée par la réaction d'isomérisation du formiate de méthyle en présence d'iridium a été proposée dans le brevet français FR2 746 794 et la demande internationale WO 97/35829.

Parallèlement, il a été proposé dans le brevet FR2 746 795 et la demande internationale WO 97/35828 un procédé de préparation d'acide acétique et/ou d'acétate de méthyle mettant en oeuvre simultanément la réaction d'isomérisation du formiate de méthyle et la réaction de carbonylation du méthanol.

Ces différents procédés de production d'acide acétique sont en général mis en oeuvre en continu dans des installations comprenant essentiellement trois zones. La première correspond à la zone de réaction proprement dite, comprenant un réacteur sous pression dans lequel la carbonylation et/ou l'isomérisation sont effectuées en phase liquide. La seconde est constituée par une zone de séparation de l'acide formé. Cette opération est réalisée par vaporisation partielle du mélange réactionnel dans un appareil appelé flash où la pression est maintenue plus faible que dans le réacteur. La partie vaporisée est ensuite envoyée dans une troisième zone où l'acide acétique produit est purifié. Celle-ci comprend, par exemple, diverses colonnes de distillation dans lesquelles l'acide acétique produit est séparé de l'eau, des réactifs et des sous-produits. La partie du mélange restée sous forme liquide en sortie de la zone de vaporisation et comprenant notamment le catalyseur est recyclée au réacteur.

Il est connu de l'homme de l'art que la seconde zone des installations de production de l'acide acétique et/ou de l'acétate de méthyle est généralement le siège d'une désactivation et/ou d'une précipitation du catalyseur, et ceci quel que soit le procédé employé parmi les procédés décrits ci-dessus.

Ces phénomènes sont généralement provoqués par la faible pression partielle de monoxyde de carbone qui règne dans cette zone, phénomènes accentués par de faibles teneurs en eau. Dans le brevet US 5,237,097, une solution proposée consiste à introduire du monoxyde de carbone à l'alimentation liquide du flash afin de maintenir une pression partielle suffisante dudit composé dans le flash.

Le brevet FR 2 726 556 et la demande internationale WO 96/14286 décrivent le procédé d'injection de monoxyde de carbone dans la fraction liquide issue du flash afin de réactiver le catalyseur recyclé au réacteur.

Le brevet EP 0 616 997 et la demande divisionnaire EP 0 786 447 correspondante proposent une amélioration du procédé de fabrication de l'acide acétique par carbonylation catalysé à l'iridium, amélioration qui consiste à maintenir la teneur en eau supérieure à 0.5 % en poids dans la fraction liquide issue du flash, afin de stabiliser le catalyseur présent dans cette fraction.

L'art antérieur ne propose aucun perfectionnement relatif à la stabilisation du catalyseur dans les procédés de fabrication d'acide acétique et/ou d'acétate de méthyle par isomérisation du formiate de méthyle et éventuellement carbonylation du méthanol catalysées à l'iridium.

Les inventeurs de la présente invention ont maintenant découvert de façon tout à fait surprenante que le problème de la désactivation et de la déstabilisation du catalyseur pouvait être résolu par le maintien d'une teneur globale en acide formique et en formiate de méthyle suffisante dans la fraction liquide issue du flash et, cela, même en présence d'une teneur particulièrement basse en eau dans cette partie de l'installation, et même pour des teneurs en eau inférieures à 0.5 % en poids par rapport à la fraction liquide non vaporisée et même en quasi absence de monoxyde de carbone dans le milieu bien que, jusqu'à présent l'homme du métier considérait qu'une teneur relativement élevée en eau et en tout cas supérieure à 0,5 % était nécessaire pour assurer la stabilité du catalyseur et qu'une teneur minimale en monoxyde de carbone permettait de stabiliser le catalyseur.

Ainsi donc, l'invention concerne, selon un premier objet, un perfectionnement applicable aux procédés de fabrication d'acide acétique et/ou d'acétate de méthyle mettant en oeuvre un système catalytique à base d'iridium, ce perfectionnement étant destiné à améliorer la stabilité et/ou éviter la désactivation du catalyseur.

Ce procédé de stabilisation permet d'abaisser considérablement la teneur en eau. aussi bien dans le milieu réactionnel que dans la zone flash, ce qui constitue un avantage non négligeable sur le plan économique puisque cela permet de limiter les coûts dans l'étape de récupération ultérieure du produit formé. Cette stabilisation du catalyseur a ainsi permis de définir les conditions d'un procédé perfectionné de préparation d'acide acétique et/ou d'acétate de méthyle incluant le perfectionnement destiné à stabiliser le catalyseur et selon lequel on utilise, en outre, une teneur particulièrement réduite en eau.

Ainsi donc, selon un deuxième aspect, l'invention concerne un procédé complet de fabrication d'acide acétique et/ou d'acétate de méthyle, dans des conditions satisfaisantes à la fois en ce qui concerne la stabilisation du catalyseur et la concentration en eau, ce qui constitue un double avantage sur le plan économique par rapport aux procédés existant à l'heure actuelle.

Plus précisément, selon le premier aspect ci-dessus, l'invention concerne un procédé pour améliorer la stabilité et/ou éviter la désactivation du catalyseur dans les procédés de fabrication d'acide acétique et/ou d'acétate de méthyle selon lesquels on réalise, dans une première étape, dite étape réactionnelle, en phase liquide, en présence de monoxyde de carbone et d'un système catalytique comprenant au moins un promoteur halogéné et au moins un composé catalytique à base d'iridium, au moins une réaction d'isomérisation du formiate de méthyle et, dans une deuxième étape, dite étape flash, la vaporisation partielle du milieu réactionnel issu de la première étape dans un séparateur dit séparateur flash. Un tel procédé consiste à maintenir dans la fraction liquide non vaporisée issue dudit séparateur flash une teneur globale en acide formique et en formiate de méthyle au moins égale à 1 % en poids de ladite fraction liquide, de préférence comprise entre 1 et 50 %, de préférence entre 1 et 30 %, en poids par rapport à ladite fraction liquide.

L'invention concerne également, selon le deuxième aspect ci-dessus. un procédé complet de Fabrication d'acide acétique et/ou d'acétate de méthyle comprenant une première étape, dite étape réactionnelle, au cours de laquelle on réalise en phase liquide, en présence de monoxyde de carbone et d'un système catalytique comprenant au moins un promoteur halogéné et au moins un composé catalytique à base d'iridium, au moins une réaction d'isomérisation du formiate de méthyle, et une deuxième étape, dite étape flash de vaporisation partielle du milieu réactionnel issu de la première étape dans un séparateur dit séparateur flash. Selon ce procédé, on maintient dans la fraction liquide non vaporisée issue dudit séparateur flash une teneur globale en acide formique et en formiate de méthyle au moins égale à 1 % en poids de ladite fraction liquide.

Selon ce procédé, la teneur en eau dans la fraction liquide issue du flash sera avantageusement maintenue inférieure à 5 %, de préférence inférieure à 2 %, de préférence encore inférieure à 0.5 % en poids par rapport à ladite fraction liquide issue du flash.

Le procédé de stabilisation et de maintien de l'activité du catalyseur exposé ci-dessus aussi bien que le procédé complet de fabrication d'acide acétique et/ou d'acétate de méthyle concernent l'un et l'autre des procédés de fabrication d'acide acétique et/ou d'acétate de méthyle dans lesquels la réaction mise en oeuvre dans l'étape réactionnelle comprend nécessairement une réaction en phase liquide d'isomérisation du formiate de méthyle en présence de monoxyde de carbone et d'un système catalytique comprenant au moins un promoteur halogéné et au moins un composé catalytique à base d'iridium.

Toutefois. selon une variante avantageuse de chacun des deux aspects de l'invention, cette réaction d'isomérisation du formiate de méthyle est réalisée simultanément avec une réaction de carbonylation du méthanol, cette carbonylation du méthanol pouvant être mise en évidence par la consommation du monoxyde de carbone introduit dans l'étape réactionnelle.

Selon une autre variante avantageuse, le procédé de stabilisation et de maintien de l'activité du catalyseur de la présente invention est réalisé en contrôlant la teneur en eau dans la fraction liquide issue du flash. Cette teneur est avantageusement maintenue inférieure à 5 % en poids et de préférence inférieure à 2 % en poids par rapport à ladite fraction liquide issue du flash.

Il est même possible, comme exposé précédemment, d'obtenir des résultats tout à fait avantageux de stabilité du catalyseur en maintenant des teneurs en eau inférieures à 0.5 % en poids dans la fraction liquide issue du flash. Ceci constitue, comme exposé précédemment, un avantage considérable par rapport aux procédés de l'art antérieur.

Selon une variante particulièrement avantageuse de l'invention, le procédé de fabrication d'acide acétique et/ou d'acétate de méthyle met en oeuvre, outre la première étape dite étape réactionnelle et la deuxième étape dite étape flash, une troisième étape dite étape de purification et récupération de l'acide acétique et/ou de l'acétate de méthyle de la fraction vaporisée issue de l'étape de vaporisation partielle.

Lors de cette étape, l'acide acétique et/ou l'acétate de méthyle sont séparés des composés légers tels que l'eau l'acide formique par différents moyens connus de l'homme de l'art.

Selon une variante tout particulièrement avantageuse de l'invention, on sépare l'acide formique de l'acide acétique par distillation réactive en injectant du méthanol dans la partie inférieure de la colonne à distiller, et en soutirant l'acide acétique purifié en pied de colonne et le mélange méthanol et formiate de méthyle en tête de colonne.

Dans la description qui va suivre, on s'attachera à définir des conditions particulièrement avantageuses aussi bien dans le milieu réactionnel que dans le milieu liquide issu du flash, conditions qui s'appliquent aussi bien au procédé de stabilisation et/ou de maintien de l'activité du catalyseur qu'au procédé complet de fabrication de l'acide acétique et/ou de l'acétate de méthyle.

Dans la description qui va suivre et, sauf indication contraire, on désignera par la "réaction", l'ensemble des réactions se produisant dans la zone réactionnelle. étant bien entendu que cette notion recouvre les réactions d'isomérisation et éventuellement de carbonylation ainsi que tous les équilibres qui se produisent dans la zone réactionnelle.

Ainsi, en particulier, par température de réaction on entendra la température imposée lors de l'étape réactionnelle.

La réaction est en général mise en oeuvre à une température comprise entre 150 et 250°C. Plus particulièrement la température de réaction est comprise entre 175 et 210°C. De préférence, elle est comprise entre 175 et 200°C.

La pression totale sous laquelle est conduite la réaction est en général supérieure à la pression atmosphérique. Plus particulièrement elle est avantageusement inférieure à $200.10^5$ Pa et, de préférence, inférieure ou égale à $50.10^5$ Pa. Les pressions sont exprimées en Pascal absolus, et sont mesurées à chaud, c'est-à-dire dans les conditions de température de la réaction.

La pression partielle de monoxyde de carbone est maintenue de préférence entre $0.5.10^5$ et $15.10^5$ Pa.

La teneur globale en acide formique et en formiate de méthyle est dans le milieu réactionnel, avantageusement maintenue à une valeur au moins égale à 1 % en poids du mélange réactionnel, de préférence comprise entre 1 % et 50 % et de préférence comprise entre 1 % et 30 %.

Le système catalytique va maintenant être décrit.

Tous les composés de l'iridium solubles ou pouvant être solubilisés dans le milieu réactionnel, dans les conditions de réalisation de l'invention, peuvent être utilisés. A titre d'exemple et sans intention de se limiter, conviennent notamment à la mise en oeuvre de l'invention l'iridium à l'état métallique, les sels simples de ce métal, les oxydes ou encore les complexes de coordination.

En tant que sels simples d'iridium, on utilise habituellement les halogénures d'iridium. L'halogène est plus particulièrement choisi parmi le chlore, le brome ou l'iode, ce dernier étant préféré. Ainsi les composés comme $IrI_3$, $IrBr_3$, $IrCl_3$, $IrI_3.4H_2O$, $IrI_4$, $IrBr_3.4H_2O$ peuvent être utilisés pour la mise en oeuvre de l'invention.

Les oxydes choisis parmi $IrO_2$, $Ir_2O_3$, $xH_2O$ peuvent de même être convenablement mis en oeuvre selon l'invention.

En ce qui concerne les complexes de coordination solubles de l'iridium, les composés les plus couramment mis en oeuvre sont ceux présentant des ligands choisis parmi le monoxyde de carbone ou une combinaison monoxyde de carbone/halogène, l'halogène étant choisi parmi le chlore, le brome ou plus particulièrement l'iode. Il n'est toutefois pas exclu d'utiliser des complexes solubles d'iridium dont les ligands sont choisis parmi des composés organo phosphorés ou organo azotés, par exemple.

En tant que complexes de coordination connus de l'homme du métier, convenant particulièrement à la mise en oeuvre de l'invention, on peut citer sans intention de se limiter les composés suivants : $Ir_4(CO)_{12}$, $Ir(CO)_2I_2^-Q^+$, $Ir(CO)_2Br_2^-Q^+$, $Ir(CO)_2Cl_2^-Q^+$ : formules dans lesquelles Q peut représenter notamment l'hydrogène, les groupes $NR_4$, $PR_4$, avec R choisi parmi l'hydrogène et/ou un radical hydrocarboné.

Ces catalyseurs peuvent être obtenus par toute méthode connue de l'homme du métier. Ainsi, on pourra se reporter aux brevets EP 657 386 et EP 737 103 pour la préparation de solutions catalytiques à base d'iridium convenant à la réalisation de la présente invention.

Il est à noter que la réaction selon l'invention peut être mise en oeuvre avec un système catalytique comprenant un composé d'iridium seul mais aussi, en outre, des composés du rhodium.

Les composés à base d'iridium et de rhodium sont décrits dans le brevet EP 0 618 183.

Lorsque l'on aura recours à un système catalytique contenant du rhodium. le rapport atomique du rhodium à l'iridium pourra varier dans une large gamme comprise entre 0.01 et 99.

Généralement. la concentration dans le milieu réactionnel en iridium ou, le cas échéant, en (iridium + rhodium) est comprise entre 0,1 et 100 mmol/l, de préférence entre 1 et 20 mmol/l.

L'ajout d'un catalyseur choisi parmi les métaux du groupe VIII de la classification périodique des éléments peut être effectué avec les composés de l'iridium ou les mélanges iridium + rhodium.

Outre les composés mentionnés ci-dessus, le système catalytique selon l'invention comprend un promoteur halogéné. Celui-ci peut se présenter sous la forme d'un halogène seul, ou en combinaison avec d'autres éléments tels que, par exemple, l'hydrogène, le radical méthyle ou acétyle.

L'halogène est en général choisi parmi le chlore, le brome ou l'iode l'iode étant préféré.

A titre de composés halogénés susceptibles d'être également utilisés en tant que promoteurs, on peut citer l'iode, l'acide iodhydrique, l'iodure de méthyle, l'iodure d'acétyle.

De préférence, on utilisera l'iodure de méthyle en tant que promoteur halogéné.

Selon une variante de l'invention, le promoteur halogéné est introduit dans le mélange réactionnel, partiellement ou totalement, sous la forme d'un précurseur. Dans un tel cas, ledit précurseur se présente généralement sous la forme d'un composé susceptible de libérer dans le milieu réactionnel, le radical hydrocarboné du promoteur halogéné précité, sous l'action d'un halogène ou de l'acide halogénohydrique notamment, ces derniers composés étant présents dans le milieu ou bien introduits dans ce but.

A titre d'exemples non limitatifs de précurseurs convenables, on peut citer les composés choisis parmi le méthanol, le diméthyléther, l'acétate de méthyle ou le formiate de méthyle, utilisés seuls ou en mélange.

La quantité de promoteur halogéné présent dans le mélange réactionnel est avantageusement inférieure ou égale à 20 %, rapporté au poids total dudit mélange. De préférence, la teneur en promoteur halogéné est inférieure ou égale à 15 %.

Il est à noter que si le promoteur précité est introduit partiellement ou totalement, sous la forme d'un précurseur, la quantité de précurseur ou de mélange promoteur/précurseur est telle qu'elle permette d'obtenir une quantité équivalente à celle mentionnée ci-dessus.

Outre ces composés, le milieu réactionnel contient de l'eau, de l'acide formique, du formiate de méthyle, de l'acétate de méthyle et de l'acide acétique, dans des proportions pondérales préférées définies ci-dessous, de préférence maintenues simultanément.

La teneur en eau est de préférence inférieure à 5 % en poids par rapport au milieu réactionnel, de préférence inférieure à 2 %.

La teneur en acide formique est de préférence inférieure à 15 % en poids du milieu réactionnel, de préférence inférieure à 12 %.

La teneur en formiate de méthyle est de préférence maintenue inférieure à 20 % en poids du milieu par rapport au milieu réactionnel.

Selon un mode particulier de l'invention, la teneur en acétate de méthyle est inférieure à 40 % en poids, de préférence inférieure à 20 %.

La teneur en acide acétique n'est pas inférieure à 25 % dans le milieu réactionnel.

Le procédé pour améliorer la stabilité et/ou éviter la désactivation du catalyseur ainsi que le procédé de préparation d'acide acétique et/ou d'acétate de méthyle selon l'invention peuvent être mis en oeuvre en présence d'iodures sous forme soluble dans le milieu réactionnel. Les iodures peuvent être introduits en tant que tels dans le milieu réactionnel mais aussi sous la forme de composés susceptibles de former des iodures solubles.

Par iodures, on entend des espèces ioniques, c'est-à-dire ne comprenant pas les iodures covalents (tels que notamment le promoteur halogéné) ni l'acide iodhydrique.

Ainsi, les iodures introduits dans ledit milieu, en tant que tels, sont choisis parmi les iodures minéraux ou organiques.

A titre d'iodures minéraux, on peut citer principalement les iodures de métaux alcalino-terreux ou alcalins, ces derniers étant préférés. On peut citer parmi ceux-ci l'iodure de potassium, l'iodure de lithium, l'iodure de sodium.

A titre d'iodures organiques, on peut citer les composés organiques, comprenant au moins un groupe organo-phosphoré et/ou au moins un groupe organo-azoté, réagissant avec des composés à base d'iode, pour donner des espèces ioniques renfermant cet halogène. A titre d'exemple, on peut mentionner l'iodure de tétraphényl phosphonium, l'iodure de N-méthyltriéthyl ammonium.

A titre de composés susceptibles de former des iodures solubles dans le milieu réactionnel, on peut citer par exemple les carboxylates, les hydroxydes de métaux alcalins ou alcalino-terreux, tels que l'acétate de lithium, la potasse, la soude notamment.

En outre, il est à noter que les iodures peuvent avoir d'autres origines que celles indiquées ci-dessus.

Ainsi, ces composés peuvent provenir d'impuretés comme les métaux alcalins ou alcalino-terreux, impuretés présentes dans les matières premières employées pour préparer la solution catalytique.

Les iodures peuvent de même provenir des métaux de corrosion apparaissant pendant la réaction.

Le procédé pour améliorer la stabilité et/ou éviter la désactivation du catalyseur ainsi que le procédé de préparation d'acide acétique et/ou d'acétate de méthyle selon l'invention sont, de préférence, mis en oeuvre en présence d'une teneur en métaux de corrosion inférieure à quelques centaines de ppm, de préférence inférieure à 200 ppm. Les métaux de corrosion sont notamment le fer, le nickel, le chrome, le molybdène et le zirconium. La teneur en métaux de corrosion dans le milieu réactionnel est maintenue par les méthodes connues de l'homme du métier, comme par exemple la précipitation sélective, l'extraction liquide liquide, le passage sur des résines échangeuses d'ions.

Les conditions dans la zone de flash vont maintenant être décrites.

La température est avantageusement maintenue entre 80°C et 200°C, la pression totale entre 0 et 20.10$^5$ Pa absolus.

Les composés présents dans la phase liquide issue du flash sont identiques à ceux contenus dans le milieu réactionnel et décrits précédemment.

La caractéristique principale de l'invention réside dans le maintien d'une teneur globale en acide formique et en formiate de méthyle au moins égale à 1 % en poids de la fraction liquide issue du flash, de préférence comprise entre 1 % et 50 %, et de préférence comprise entre 1 % et 30 %.

Exprimées en pourcentages pondéraux par rapport à la fraction liquide non vaporisée issue du flash, les proportions des différents constituants maintenues de préférence simultanément sont avantageusement les suivants :

- la teneur en promoteur halogéné est inférieure à 20 %, de préférence inférieure à 15%,
- la teneur en eau est inférieure à 5 %, de préférence inférieure à 2 %, et selon un mode de réalisation particulièrement avantageux, la stabilité du catalyseur est assurée pour une teneur en eau inférieure à 0,5 %.
- la teneur en acide formique est inférieure à 15 %, de préférence inférieure à 12 %,
- la teneur en formiate de méthyle est maintenue inférieure à 20 %,
- selon un mode particulier de l'invention, la teneur en acétate de méthyle est inférieure à 40 %, de préférence inférieure à 20 %,
- la teneur en acide acétique n'est pas inférieure à 25 %.

La fraction liquide non vaporisée issue du flash peut contenir des iodures sous forme de composés ioniques solubles dans ladite fraction (se reporter à leur description dans le milieu réactionnel).

La teneur en monoxyde de carbone contenu dans le flash est non nulle. Le monoxyde de carbone peut provenir du milieu réactionnel à vaporiser sous forme de CO dissous et entraîné. Il peut, en plus, être injecté directement dans la fraction liquide issue du flash et recyclée au réacteur. En tout état de cause, la pression partielle de monoxyde de carbone dans la zone de flash est inférieure à la pression partielle de monoxyde de carbone maintenue dans la zone réactionnelle.

Dans le procédé de fabrication de l'acide acétique et/ou de l'acétate de méthyle décrit ci-dessus, ces deux produits contenus dans la phase gazeuse issue du flash sont séparés des composés légers, de l'eau, de l'acide formique et des autres impuretés, par exemple par distillation fractionnée dans une ou plusieurs colonnes à distiller. Certains de ces composés peuvent être alors recyclés au réacteur.

Dans un mode préféré de réalisation, l'acide formique est séparé de l'acide acétique par distillation réactive en injectant du méthanol dans la partie inférieure de la colonne à distiller, le formiate de méthyle préférentiellement formé est recyclé au réacteur et l'acide acétique ainsi purifié est soutiré en fond de la colonne.

D'une façon générale, l'invention s'applique avantageusement à un procédé de fabrication mis en oeuvre en continu.

**EXEMPLES**

**I Tests de stabilité**

I-1. Equipement utilisé

Tous les essais sont réalisés en tube transparent tenant à de hautes pressions, présentant une longueur de 16 cm et un diamètre intérieur de 0,7 cm ; soit un volume total de 6 ml.

Ce tube est équipé :

- d'une alimentation en gaz permettant de purger le ciel gazeux du tube, en général avec du monoxyde de carbone,
- d'un chauffage du tube par four électrique avec régulation de température,
- d'une agitation par table oscillante.

I-2. Préparation de la masse réactionnelle

* Préparation de la solution catalytique par mise en solution d'iodure d'iridium dans un mélange d'acide iodhydrique et d'acide acétique, dans un réacteur agité par chauffage à 150°C sous pression de monoxyde de carbone de

50.10$^5$ Pa (50 bars) pendant 4 h. La solution catalytique ainsi obtenue titre environ 2,6 % en iridium (soit 26 000 ppm ou mg/kg).

  \*   Préparation de la masse réactionnelle pour le test de stabilité, par pesée et mélange des différents constituants :

- acide acétique, acétate de méthyle, iodure de méthyle,
- solution catalytique en tenant compte des quantités d'acide acétique apportées par celle-ci,
- et pour les essais 1 à 10, acide formique et formiate de méthyle.

  \*   La masse réactionnelle (composition initiale) ainsi préparée est analysée.

I-3. Mode opératoire général

Dans le tube, on charge 4 g de masse réactionnelle, on agite le tube, on purge 3 fois avec le gaz approprié et on laisse ensuite sous pression de ce gaz à 2,2.10$^5$ Pa (2,2 bars absolus).

Ce gaz est le monoxyde de carbone pour les essais 1, 2, 3, 4, 5, 7, 8 A, B, C, D ; l'air pour les essais 6, 9 et 10. On agite encore 20 secondes puis on isole le tube. On pèse le tube pour vérifier l'absence de fuite. On le place dans le four électrique et on chauffe le temps désiré, à la température désirée (130 ou 150°C), puis on arrête le chauffage et on refroidit le tube. On pèse à nouveau le tube pour vérifier l'absence de fuite, dans ce cas le test est validé. On effectue ensuite un examen visuel du tube et de son contenu : aspect, coloration, dépôt/précipité. On prélève les échantillons nécessaires pour effectuer les analyses sur les masses réactionnelles, avant (état initial) et après le déroulement du test de stabilité (état final).

I-4. Méthodes d'analyse

- La concentration en iridium est mesurée par Spectroscopie Induite Couplée au Plasma (ICP, pour Inductive Coupled Plasma Spectroscopy)
- la concentration en eau est déterminée par la méthode de Karl Fischer
- la composition des produits organiques est mesurée par chromatographie en phase gazeuse
- la stabilité de l'iridium exprimée en pourcentage en solution est calculée selon :

$$\frac{[Ir]\ finale}{[Ir]initiale} \times 100 \text{ ; les stabilités iridium sont à } \pm 10\ \%.$$

I-5 Résultats

I-5. a) Tests de stabilité à 130°C

Exemples 1 à 6 et A à D à 130°C et Tableaux A et B des résultats des tests de stabilité

Les essais 1 à 6 correspondent à des essais selon l'invention. Les conditions et les résultats de ces essais sont donnés dans le tableau A ci-après.

Les essais A à D sont des exemples comparatifs et ne correspondent pas à des essais selon l'invention. Les conditions et résultats sont donnés dans le tableau B ci-après.

Dans les tableaux A et B, les résultats sont donnés pour chaque essai et pour chaque état initial et final. On précise dans ces tableaux :

- la composition du mélange réactionnel en % pondéral en acide acétique (AcOH), acide formique (HCOOH), acétate de méthyle (AcOMe), formiate de méthyle (HCOOMe). iodure de méthyle (ICH$_3$), eau (H$_2$O)
- la teneur en iridium en ppm (mg/kg)
- la pression absolue du gaz (PCO) en 10$^5$ Pa (ou bar)
- la durée du chauffage à 130°C
- le pourcentage d'iridium en solution après la période de chauffage.

Analyse des tableaux A et B - Exploitation des résultats des essais 1 à 6

Dans tout ce qui va suivre. on appellera "composés formyles". l'ensemble acide formique + formiate de méthyle.

Exemples 1 et A, essais à 0.4 % d'eau

Pour des teneurs initiales sensiblement identiques en acétate et iodure de méthyle (11 et 2 %) et en présence de 0.4 % d'eau, après 120 min à 130°C, la stabilité de l'iridium dans l'exemple 1 est de 95 % contre 31 % dans l'exemple comparatif A.
La présence initiale de 13 % de "composés formyles" stabilise fortement le catalyseur.

Exemples 2 et B, essais en l'absence d'eau

Dans l'exemple 2, les teneurs initiales sont proches de celles de l'exemple 1 : en l'absence d'eau, après 15 min à 130°C, il n'y a pas de perte d'iridium. Dans des conditions sensiblement identiques, en l'absence d'eau et des "composés formyles", l'exemple comparatif B voit la stabilité du catalyseur chuter à 72 %.

Exemple 3 : essai en l'absence d'eau

Dans l'exemple 3, la diminution de la teneur initiale des "composés formyles" à 4.7 %, en l'absence d'eau, permet de conserver une remarquable stabilité de l'iridium après 15 min à 130°C (100%).

Exemple 4: essai à 16,7 % de "composés formyles"

A l'inverse de l'essai 3. la teneur initiale en "composés formyles" est augmentée à 16.7 %. Après 60 min à 130°C, la stabilité de l'iridium est assurée à 96 % en présence de seulement 0.3 % d'eau.

Exemple 5, C et D, essais à teneur en iodure de méthyle élevée

Ces 3 essais sont réalisés à 130°C, 120 min sous une pression de monoxyde de carbone absolue de $2.2.10^5$ Pa. conditions générales.
Dans l'exemple 5, en présence initiale de 5,7 % de "composés formyles", de 5,1 % d'iodure de méthyle, la stabilité de l'iridium est de 96 %.
En l'absence de "composés formyles" et pour des teneurs en eau et acétate de méthyle sensiblement équivalentes, les exemples comparatifs C et D montrent :

- qu'en présence de faible teneur en iodure de méthyle initiale à 1.3 % (essai C), la stabilité de l'iridium est fortement dégradée (27 %).
- l'augmentation de la teneur en iodure de méthyle initiale à 9 % (essai D) améliore la stabilité de l'iridium à 73 % (action connue de l'art antérieur) mais ne permet pas d'atteindre la stabilité de l'essai 5 (96 %).

Exemple 6 en l'absence de monoxyde de carbone

Cet essai est conduit sous air : en l'absence de CO après 60 min à 130°C, la présence initiale de 5.3 % de "composés formyles" permet de maintenir une stabilité de l'iridium de 90 % à comparer aux bilans de stabilité de l'iridium dans les essais comparatifs qui sont :

- de 31 % après 120 min (essai comparatif A) avec une composition initiale en iodure et acétate de méthyle identique à l'essai 6
- de 27 % après 120 min (essai comparatif C).

I-5. b) Tests de stabilité à 150°C

Le tableau C ci-après donne les résultats et les conditions d'essais de stabilité selon l'invention (7, 8, 9 et 10) et comparatif (E) réalisés à 150°C.

Exemples 7, 8 et E à 150°C - Tableau C des résultats des tests de stabilité

Les essais sont réalisés dans les mêmes conditions que les exemples précédents sauf que la température de chauffage est de 150°C au lieu de 130°C.

Les conditions et résultats de ces essais sont donnés dans le tableau C ci-après.

Pour des teneurs en eau sensiblement identiques entre 0,35 et 0.40 %, ces 3 essais montrent l'importance de la présence des "composés formyles" (teneurs initiales 6.3 % essai 7-13.2 % essai 8) sur la stabilité de l'iridium respectivement 93 et 99 % contre 40 % pour l'essai comparatif E réalisé en l'absence de ces mêmes composés.

Maintenant, si on regarde l'influence de l'augmentation de la température entre 130°C et 150°C, on peut comparer deux à deux les essais 3 et 7, 2 et 8. B et E pour constater une baisse de la stabilité de l'iridium (toutes choses égales par ailleurs : composition, durée)

-   très faible pour les essais selon l'invention
    100% → 93% et 100% → 99%
-   importante pour l'essai comparatif E
    72 % → 40 %

ce qui prouve bien l'influence et l'intérêt des "composés formyles" : acide formique et formiate de méthyle.

Exemples 9 et 10 à 150°C et en l'absence de monoxyde de carbone - Tableau C

Ces essais sont destinés à montrer l'influence positive d'une légère augmentation de la teneur en eau ainsi que la remarquable stabilité de l'iridium en l'absence totale de monoxyde de carbone, quand des "composés formyles" sont présents.

-   Comparée aux essais 7 et 8 (en présence de CO) et pour 15 minutes de réaction, la stabilité de l'iridium est du même ordre dans l'essai 9 réalisé en l'absence de CO (96 % contre 93 et 99 %).
-   L'augmentation de la température de 130°C (essai 6) à 150°C (essai 10) ne provoque pas la chute de la stabilité de l'iridium (90 % essai 6 contre 93 % essai 10), ces 2 essais étant réalisés sous air, pendant 60 minutes de réaction.

**TABLEAU A : RESULTATS DES TESTS DE STABILITE, ESSAIS SELON L'INVENTION**

| ESSAI N° | ETAT | AcOH | AcOMe | ICH3 | HCOOH | HCOOMe | H2O | Iridium | P°CO | DUREE | TEMPER. | STABI-LITE Ir |
|---|---|---|---|---|---|---|---|---|---|---|---|---|
| | | % | % | % | % | % | % | mg/kg (ppm) | Bar | minute | °C | % |
| 1 | INITIAL | 71 | 11,0 | 2,0 | 12,0 | 1,00 | 0,41 | 6700 | 2,2 | | | |
| | FINAL | 74 | 7,9 | 3,4 | 11,0 | 1,40 | | 6370 | | 120 | 130 | 95 |
| 2 | INITIAL | 76 | 11,0 | 1,7 | 10,0 | 1,00 | 0 | 2880 | 2,2 | | | |
| | FINAL | 76 | 10,0 | 2,0 | 10,0 | 1,20 | | 2880 | | 15 | 130 | 100 |
| 3 | INITIAL | 78 | 12,0 | 1,9 | 3,7 | 0,99 | 0 | 7015 | 2,2 | | | |
| | FINAL | 79 | 11,0 | 3,0 | 3,6 | 0,77 | | 7185 | | 15 | 130 | 100 |
| 4 | INITIAL | 70 | 11,0 | 1,8 | 15,0 | 1,70 | 0,29 | 6590 | 2,2 | | | |
| | FINAL | 71 | 8,5 | 3,0 | 14,0 | 2,10 | | 6310 | | 60 | 130 | 96 |
| 5 | INITIAL | 80 | 7,7 | 5,1 | 4,7 | 1,00 | 0,37 | 7200 | 2,2 | | | |
| | FINAL | 81 | 7,1 | 6,3 | 4,3 | 0,50 | | 6895 | | 120 | 130 | 96 |
| 6 | INITIAL | 78 | 11,0 | 1,8 | 4,4 | 0,93 | 0,41 | 6760 | 0 (AIR) | | | |
| | FINAL | 79 | 11,0 | 3,1 | 4,6 | 0,79 | | 6090 | | 60 | 130 | 90 |

EP 1 218 329 B1

## TABLEAU B : RESULTATS DES TESTS DE STABILITE, ESSAIS COMPARATIFS

EP 1 218 329 B1

| ESSAI N° | ETAT | AcOH | AcOMe | ICH3 | HCOOH | HCOOMe | H2O | Iridium | P CO | DUREE | TEMPER. | STABI-LITE Ir |
|---|---|---|---|---|---|---|---|---|---|---|---|---|
| | | % | % | % | % | % | % | mg/kg (ppm) | Bar | minute | °C | % |
| A | INITIAL | 83 | 11,0 | 2,0 | 0 | 0 | 0,40 | 6725 | 2,2 | | | |
| | FINAL | 84 | 11,0 | 3,0 | 0 | 0 | | 2105 | | 120 | 130 | 31 |
| B | INITIAL | 89 | 7,3 | 0,3 | 0 | 0 | 0 | 7070 | 2,2 | | | |
| | FINAL | 90 | 6,9 | 1,0 | 0 | 0 | | 5090 | | 15 | 130 | 72 |
| C | INITIAL | 87 | 7,6 | 0,2 | 0 | 0 | 0,39 | 6745 | 2,2 | | | |
| | FINAL | 90 | 7,2 | 1,3 | 0 | 0 | | 1840 | | 120 | 130 | 27 |
| D | INITIAL | 83 | 7,4 | 9,0 | 0 | 0 | 0,38 | 7015 | 2,2 | | | |
| | FINAL | 83 | 7,2 | 9,2 | 0 | 0 | | 5090 | | 120 | 130 | 73 |

## TABLEAU C : RESULTATS DES TESTS DE STABILITE, INFLUENCE DE LA TEMPERATURE 150°C

| ESSAI N° | ETAT | AcOH | AcOMe | ICH3 | HCOOH | HCOOMe | H2O | Iridium | P CO | DUREE | TEMPER. | STABI-LITE Ir |
|---|---|---|---|---|---|---|---|---|---|---|---|---|
| | | % | % | % | % | % | % | mg/kg (ppm) | Bar | min | °C | % |
| 7 | INITIAL | 77 | 12,0 | 2,1 | 5,2 | 1,10 | 0,39 | 6830 | 2,2 | | | |
| | FINAL | 79 | 11,0 | 3,3 | 4,8 | 0,83 | | 6320 | | 15 | 150 | 93 |
| 8 | INITIAL | 72 | 11,0 | 2,1 | 12,0 | 1,20 | 0,40 | 6940 | 2,2 | | | |
| | FINAL | 73 | 9,7 | 3,4 | 10,0 | 1,60 | | 6850 | | 15 | 150 | 99 |
| E comparatif | INITIAL | 88 | 7,8 | 0,3 | 0 | 0 | 0,35 | 6805 | 2,2 | | | |
| | FINAL | 89 | 7,4 | 1,1 | 0 | 0 | | 2730 | | 15 | 150 | 40 |
| 9 | INITIAL | 77 | 12 | 2,2 | 4,7 | 0,95 | 2,2 | 7285 | 0(AIR) | | | |
| | FINAL | 77 | 11 | 2,6 | 4,8 | 0,86 | | 6985 | | 15 | 150 | 96 |
| 10 | INITIAL | 77 | 12 | 2,2 | 4,7 | 0,95 | 2,2 | 7285 | 0(AIR) | | | |
| | FINAL | 78 | 11 | 3,2 | 4,8 | 0,81 | | 6740 | | 60 | 150 | 93 |

EP 1 218 329 B1

II - Exemple de procédé complet selon l'invention

Exemple 11

Réaction d'isomérisation et de carbonylation avec recyclage de catalyseur

Dans un autoclave en Hastelloy® B2 contenant de la solution catalytique préparée selon la méthode décrite dans les exemples précédents, on injecte en continu les différents composants du mélange réactionnel : acide acétique, formiate de méthyle, méthanol, acétate de méthyle, iodure de méthyle, et éventuellement eau. Les flux sortant du réacteur sont dirigés dans une zone où une fraction contenant l'acide acétique produit est vaporisée. La fraction non vaporisée contenant le catalyseur est recyclée au réacteur. La fraction vaporisée est condensée et représente les effluents liquides.

On maintient dans cet essai, dans la fraction liquide non vaporisée issue du flash, une concentration totale en acide formique et formiate de méthyle de 6,5 % et une teneur en eau de 1%.

La composition du mélange réactionnel en régime stabilisé, déterminée par dosage en chromatographie en phase vapeur d'un échantillon prélevé du milieu réactionnel, exprimée en pourcentages massiques est la suivante :

| | |
|---|---|
| eau | 1,3% |
| méthanol | 0.1% |
| acétate de méthyle | 16.1 % |
| iodure de méthyle | 9.7 % |
| acide formique | 4,3 % |
| formiate de méthyle | 1,6 % |
| acide acétique | complément à 100 % |
| la concentration en iridium est de | 2050 mg/kg (ppm). |

La température est maintenue à 190°C +/- 0,5°C.

La pression totale du réacteur est maintenue à 2.4 MPa +/- 20 kPa (24 bars).

La pression partielle de monoxyde de carbone est maintenue constante à une valeur de 1.05 MPa (10.5 bars) ; le CO utilisé est de pureté supérieure à 99%.

Le calcul de la vitesse de formation de l'acide acétique par les deux réactions d'isomérisation du formiate de méthyle et de carbonylation du méthanol est effectué grâce aux bilans sorties/entrées réalisés sur les effluents liquides de la zone de vaporisation, collectés pendant une durée donnée (comprise entre les 40e et 43e heures de fonctionnement), par rapport aux flux des composés injectés pendant le même intervalle de temps, après que le régime chimique a été stabilisé. Le calcul de la vitesse de carbonylation est fait d'après la consommation de monoxyde de carbone par la réaction de carbonylation (bilan entrée/sortie).

On obtient une vitesse d'isomérisation de 1,4 mol.h$^{-1}$l$^{-1}$ en acide acétique formé, et une vitesse de carbonylation de 16,4 mol.h$^{-1}$l$^{-1}$ en acide acétique formé. L'acide acétique se trouve sous la forme d'acide acétique et d'acétate de méthyle.

Le TOF (abréviation anglaise de Turnover Frequency), calculé comme le rapport de la vitesse totale des 2 réactions (17,8 mol.h$^{-1}$.l$^{-1}$) à la concentration en catalyseur du milieu réactionnel (0,01067 mol.l$^{-1}$) s'élève à 1 670 h$^{-1}$.

Dans cet essai d'une durée globale de fonctionnement de 100 heures, entre le début et la fin de l'essai, il n'a pas été constaté de perte de catalyseur par précipitation ou désactivation. Ce fait est établi à partir des analyses régulières des concentrations en iridium du milieu réactionnel, de la fraction vaporisée et de la fraction liquide non vaporisée (recyclée vers le réacteur) issues de la zone de flash.

**Revendications**

**1.** Procédé pour améliorer la stabilité et/ou éviter la désactivation du catalyseur dans les procédés de fabrication d'acide acétique et/ou d'acétate de méthyle selon lesquels on réalise, dans une première étape, dite étape réactionnelle, en phase liquide, en présence de monoxyde de carbone et d'un système catalytique comprenant au moins un promoteur halogéné et au moins un composé catalytique à base d'iridium, au moins une réaction d'isomérisation du formiate de méthyle et, dans une deuxième étape, dite étape flash, la vaporisation partielle du milieu réactionnel issu de la première étape dans un séparateur dit séparateur flash, **caractérisé en ce qu'**il consiste à maintenir dans la fraction liquide non vaporisée issue dudit séparateur flash une teneur globale en acide formique et en formiate de méthyle au moins égale à 1 % en poids de ladite fraction liquide, de préférence comprise entre 1 et 50 %, de préférence entre 1 et 30 %, en poids par rapport à ladite fraction liquide.

**2.** Procédé selon la revendication 1, **caractérisé en ce qu'**il est appliqué à un procédé de fabrication d'acide acétique et/ou d'acétate de méthyle réalisé en continu.

**3.** Procédé de fabrication d'acide acétique et/ou d'acétate de méthyle comprenant une première étape, dite étape réactionnelle, au cours de laquelle on réalise en phase liquide, en présence de monoxyde de carbone et d'un système catalytique comprenant au moins un promoteur halogéné et au moins un composé catalytique à base d'iridium, au moins une réaction d'isomérisation du formiate de méthyle, et une deuxième étape, dite étape flash de vaporisation partielle du milieu réactionnel issu de la première étape dans un séparateur dit séparateur flash, **caractérisé en ce que** l'on maintient dans la fraction liquide non vaporisée issue dudit séparateur flash une teneur globale en acide formique et en formiate de méthyle au moins égale à 1 % en poids de ladite fraction liquide.

**4.** Procédé selon la revendication 3, **caractérisé en ce qu'**il s'agit d'un procédé de fabrication d'acide acétique et/ou d'acétate de méthyle réalisé en continu.

**5.** Procédé selon l'une des revendications 1 à 4, **caractérisé en ce que** ladite première étape comprend en outre une réaction de carbonylation du méthanol.

**6.** Procédé selon l'une des revendications 1 à 5, **caractérisé en ce que** l'on maintient dans ladite fraction liquide une teneur en eau inférieure à 5 % en poids, de préférence inférieure à 2 % en poids par rapport à ladite fraction liquide issue du flash.

**7.** Procédé selon la revendication 6, **caractérisé en ce que** ladite teneur en eau est maintenue inférieure à 0,5 % en poids par rapport à la fraction liquide issue du flash.

**8.** Procédé selon l'une des revendications 1 à 7, **caractérisé en ce que** ladite deuxième étape de vaporisation partielle est suivie d'une troisième étape de purification et récupération de l'acide acétique et/ou de l'acétate de méthyle de la fraction vaporisée issue de ladite étape de vaporisation partielle.

**9.** Procédé selon la revendication 8, **caractérisé en ce que**, dans la troisième étape de purification et récupération, l'acide formique est séparé de l'acide acétique par distillation réactive en injectant du méthanol dans la partie inférieure de la colonne à distiller, et en soutirant l'acide acétique purifié en pied de colonne et le mélange méthanol et formiate de méthyle en tête de colonne.

**10.** Procédé selon l'une des revendications 1 à 9, **caractérisé en ce que** l'on maintient le promoteur halogéné dans le milieu réactionnel de la première étape à une concentration inférieure ou égale à 20 % en poids.

**11.** Procédé selon l'une des revendications 1 à 10, **caractérisé en ce que** l'on maintient la teneur en acide formique dans le milieu réactionnel de la première étape à une valeur inférieure à 15 % en poids.

**12.** Procédé selon l'une des revendications 1 à 11, **caractérisé en ce que** l'on maintient la teneur en formiate de méthyle dans le milieu réactionnel de la première étape à une valeur inférieure à 20 % en poids.

**13.** Procédé selon l'une des revendications 1 à 12, **caractérisé en ce que** l'on maintient la teneur en acétate de méthyle dans le milieu réactionnel de la première étape à une valeur inférieure à 40 % en poids.

**14.** Procédé selon l'une des revendications 1 à 13, **caractérisé en ce qu'**on maintient la teneur en acide acétique dans le milieu réactionnel de la première étape à une valeur qui n'est pas inférieure à 25 % en poids.

**15.** Procédé selon l'une des revendications 1 à 14, **caractérisé en ce que** ledit promoteur halogéné est choisi parmi les composés iodés et leurs précurseurs.

**16.** Procédé selon la revendication 15, **caractérisé en ce que** ledit promoteur halogéné est choisi dans le groupe constitué de l'iode, de l'iodure de méthyle, de l'acide iodhydrique et de l'iodure d'acétyle.

**17.** Procédé selon la revendication 16, **caractérisé en ce que** ledit promoteur halogéné est de l'iodure de méthyle.

**18.** Procédé selon l'une des revendications 1 à 17, **caractérisé en ce que** le système catalytique comprend en outre un composé catalytique à base de rhodium.

**19.** Procédé selon l'une des revendications 1 à 18, **caractérisé en ce que** la concentration globale en composé(s) catalytique(s) est comprise entre 0,1 et 100 mmol/l dans le milieu réactionnel de la première étape.

**20.** Procédé selon l'une des revendications 1 à 19, **caractérisé en ce qu'**on maintient dans le milieu réactionnel de la première étape des iodures sous forme d'un composé ionique soluble dans ledit milieu.

**21.** Procédé selon l'une des revendications 1 à 20, **caractérisé en ce que**, dans l'étape réactionnelle, la température est maintenue entre 150 et 250°C, de préférence entre 175° et 210°C, et la pression à une valeur comprise entre 0 et $200.10^5$ Pa absolus, de préférence inférieure à $50.10^5$ Pa absolus.

**22.** Procédé selon l'une des revendications 1 à 21, **caractérisé en ce que**, dans l'étape de vaporisation partielle, la température est maintenue entre 80 et 200°C et la pression entre 0 et $20.10^5$ Pa absolus.

**Patentansprüche**

**1.** Verfahren zur Verbesserung der Stabilität und/oder zur Vermeidung der Desaktivierung des Katalysators in Verfahren zur Herstellung von Essigsäure und/oder Methylacetat, bei denen man
in einer ersten Stufe, einer so genannten Reaktionsstufe, in flüssiger Phase in Gegenwart von Kohlenmonoxid und eines katalytischen Systems, das mindestens einen halogenierten Promotor und mindestens eine katalytische Verbindung auf Iridiumbasis umfasst, mindestens eine Reaktion zur Isomerisierung des Methylformiats durchführt, und in einer zweiten Stufe, einer so genannten Flash-Stufe, die partielle Verdampfung des Reaktionsmediums, das aus der ersten Stufe stammt, in einem Separator, einem so genannten Flash-Separator, durchführt, **dadurch gekennzeichnet, dass** das Verfahren darin besteht, dass man in der flüssigen, nicht-verdampften Fraktion, die aus dem Flash-Separator stammt, einen Gesamtgehalt von Ameisensäure und Methylformiat von mindestens 1 Gew.-%, vorzugsweise von 1 bis 50 Gew.-%, besonders bevorzugt von 1 bis 30 Gew.-%, bezogen auf das Gewicht der flüssigen Fraktion, aufrechterhält.

**2.** Verfahren nach Anspruch 1, **dadurch gekennzeichnet, dass** man es anwendet auf ein kontinuierlich durchgeführtes Verfahren zur Herstellung von Essigsäure und/oder Methylacetat.

**3.** Verfahren zur Herstellung von Essigsäure und/oder Methylacetat, das umfasst
eine erste Stufe, eine so genannte Reaktionsstufe, in deren Verlauf man in flüssiger Phase in Gegenwart von Kohlenmonoxid und eines katalytischen Systems, das mindestens einen halogenierten Promotor und mindestens eine katalytische Verbindung auf Basis von Iridium umfasst, mindestens eine Reaktion zur Isomerisierung von Methylformiat durchführt, und
eine zweite Stufe, eine so genannte Flash-Stufe, zur partiellen Verdampfung des Reaktionsmediums, das aus der ersten Stufe stammt, in einem Separator, einem so genannten Flash-Separator, **dadurch gekennzeichnet, dass** man in der nicht verdampften flüssigen Fraktion, die aus dem Flash-Separator stammt, einen Gesamtgehalt von Ameisensäure und Methylformiat von mindestens 1 Gew.-%, bezogen auf das Gewicht der flüssigen Fraktion, aufrechterhält.

**4.** Verfahren nach Anspruch 3, **dadurch gekennzeichnet, dass** es sich dabei um ein kontinuierliches Verfahren zur Herstellung von Essigsäure und/oder Methylacetat handelt.

**5.** Verfahren nach einem der Ansprüche 1 bis 4, **dadurch gekennzeichnet, dass** die erste Stufe außerdem eine Reaktion zur Carbonylierung von Methanol umfasst.

**6.** Verfahren nach einem der Ansprüche 1 bis 5, **dadurch gekennzeichnet, dass** man in der flüssigen Fraktion einen Wassergehalt von < 5 Gew.-%, vorzugsweise von < 2 Gew.-%, bezogen auf das Gewicht der aus der Flash-Stufe stammenden flüssigen Fraktion, aufrechterhält.

**7.** Verfahren nach Anspruch 6, **dadurch gekennzeichnet, dass** der Wassergehalt bei weniger als 0,5 Gew.-% gehalten wird, bezogen auf die aus der Flash-Stufe stammende flüssige Fraktion.

8. Verfahren nach einem der Ansprüche 1 bis 7, **dadurch gekennzeichnet, dass** auf die zweite Stufe der partiellen Verdampfung eine dritte Stufe der Reinigung und Abtrennung der Essigsäure und/oder des Methylacetats von der verdampften Fraktion, die aus der Stufe der partiellen Verdampfung stammt, folgt.

9. Verfahren nach Anspruch 8, **dadurch gekennzeichnet, dass** in der dritten Reinigungs- und Abtrennungsstufe die Ameisensäure durch reaktive Destillation von der Essigsäure getrennt wird, indem man Methanol in den unteren Abschnitt der Destillationskolonne injiziert und die gereinigte Essigsäure am Fuß der Kolonne und die Mischung von Methanol und Methylformiat am Kopf der Kolonne abzieht.

10. Verfahren nach einem der Ansprüche 1 bis 9, **dadurch gekennzeichnet, dass** man den halogenierten Promotor in dem Reaktionsmedium der ersten Stufe bei einer Konzentration von ≤ 20 Gew.-% hält.

11. Verfahren nach einem der Ansprüche 1 bis 10, **dadurch gekennzeichnet, dass** man den Ameisensäure-Gehalt in dem Reaktionsmedium der ersten Stufe bei einem Wert von < 15 Gew.-% hält.

12. Verfahren nach einem der Ansprüche 1 bis) 11, **dadurch gekennzeichnet, dass** man den Methylformiat-Gehalt in dem Reaktionsmedium der ersten Stufe bei einem Wert von < 20 Gew.-% hält.

13. Verfahren nach einem der Ansprüche 1 bis 12, **dadurch gekennzeichnet, dass** man den Methylacetat-Gehalt in dem Reaktionsmedium der ersten Stufe bei einem Wert unter 40 Gew.-% hält.

14. Verfahren nach einem der Ansprüche 1 bis 13, **dadurch gekennzeichnet, dass** man den Essigsäure-Gehalt in dem Reaktionsmedium der ersten Stufe bei einem Wert hält, der nicht unter 25 Gew.-% liegt.

15. Verfahren nach einem der Ansprüche 1 bis 14, **dadurch gekennzeichnet, dass** der halogenierte Promotor ausgewählt wird unter den iodierten Verbindungen und ihren Vorläufern.

16. Verfahren nach Anspruch 15, **dadurch gekennzeichnet, dass** der halogenierte Promotor ausgewählt wird aus der Gruppe, die besteht aus Iod, Methyliodid, Iodwasserstoffsäure und Acetyliodid.

17. Verfahren nach Anspruch 16, **dadurch gekennzeichnet, dass** es sich bei dem halogenierten Promotor um Methyliodid handelt.

18. Verfahren nach einem der Ansprüche 1 bis 17, **dadurch gekennzeichnet, dass** das katalytische System außerdem eine katalytische Verbindung auf Basis von Rhodium umfasst.

19. Verfahren nach einem der Ansprüche 1 bis 18, **dadurch gekennzeichnet, dass** die Gesamtkonzentration an katalytischer (katalytischen) Verbindung(en) in dem Reaktionsmedium der ersten Stufe zwischen 0,1 und 100 mmol/l liegt.

20. Verfahren nach einem der Ansprüche 1 bis 19, **dadurch gekennzeichnet, dass** man in dem Reaktionsmedium der ersten Stufe Iodide in Form einer in dem Medium löslichen ionischen Verbindung aufrechterhält.

21. Verfahren nach einem der Ansprüche 1 bis 20, **dadurch gekennzeichnet, dass** man in der Reaktionsstufe die Temperatur zwischen 150 und 250 °C, vorzugsweise zwischen 175 und 210 °C, und den Druck bei einem Wert zwischen 0 und $200.10^5$ Pa abs., vorzugsweise bei einem Wert unter $50.10^5$ Pa abs. hält.

22. Verfahren nach einem der Ansprüche 1 bis 21, **dadurch gekennzeichnet, dass** in der partiellen Verdampfungsstufe die Temperatur zwischen 80 und 200 °C und der Druck zwischen 0 und $20.10^5$ Pa abs. gehalten werden.

**Claims**

1. A process for improving the stability and/or preventing the deactivation of the catalyst in processes of manufacture of acetic acid and/or of methyl acetate according to which processes, in a first step, referred to as the reaction step, at least one methyl formate isomerisation reaction is carried out in liquid phase, in the presence of carbon monoxide and of a catalytic system comprising at least one halogenated promoter and at least one iridium-based catalytic compound, and, in a second step, referred to as the flash step, the partial vaporisation of the reaction medium originating from the first step is carried out in a separator referred to as the flash separator, **characterised in that** it consists in maintaining an overall content of formic acid and of methyl formate at least equal to 1 % by weight of said liquid fraction, preferably between 1 and 50 %, preferably between 1 and 30 %, by weight with respect to said liquid fraction, in the non-vaporised liquid fraction originating from said flash separator.

2. The process according to claim 1, **characterised in that** it is applied to a process of manufacture of acetic acid and/or of methyl acetate which is carried out continuously.

3. A process of manufacture of acetic acid and/or of methyl acetate comprising a first step, referred to as the reaction step, during which at least one methyl formate isomerisation reaction is carried out, in liquid phase, in the presence of carbon monoxide and of a catalytic system comprising at least one halogenated promoter and at least one iridium-based catalytic compound, and a second step, referred to as the flash step of partial vaporisation of the reaction medium originating from the first step, is carried out in a separator referred to as the flash separator, **characterised in that** an overall content of formic acid and of methyl formate at least equal to 1 % by weight of said liquid fraction is maintained in the non-vaporised liquid fraction originating from said flash separator.

4. The process according to claim 3, **characterised in that** said process is a process of manufacture of acetic acid and/or of methyl acetate carried out continuously.

5. The process according to one of claims 1 to 4, **characterised in that** said first step further comprises a methanol carbonylation reaction.

6. The process according to one of claims 1 to 5, **characterised in that** a water content of less than 5 % by weight, preferably of less than 2 % by weight with respect to said liquid fraction originating from the flash, is maintained in said liquid fraction.

7. The process according to claim 6, **characterised in that** said water content is maintained at less than 0.5 % by weight with respect to the liquid fraction originating from the flash.

8. The process according to one of claims 1 to 7, **characterised in that** said second step of partial vaporisation is followed by a third step of purification and recovery of the acetic acid and/or of the methyl acetate from the vaporised fraction originating from said step of partial vaporisation.

9. The process according to claim 8, **characterised in that**, in the third step of purification and recovery, the formic acid is separated from the acetic acid by reactive distillation by injecting methanol into the lower part of the distilling column, and by removing the purified acetic acid at the bottom of the column and the methanol and methyl formate mixture at the head of the column.

10. The process according to one of claims 1 to 9, **characterised in that** the halogenated promoter is maintained in the reaction medium of the first step at a concentration of less than or equal to 20 % by weight.

11. The process according to one of claims 1 to 10, **characterised in that** the content of formic acid is maintained in the reaction medium of the first step at a value of less than 15 % by weight.

12. The process according to one of claims 1 to 11, **characterised in that** the methyl formate content is maintained in the reaction medium of the first step at a value of less than 20 % by weight.

13. The process according to one of claims 1 to 12, **characterised in that** the methyl acetate content is maintained in the reaction medium of the first step at a value of less than 40 % by weight.

14. The process according to one of claims 1 to 13, **characterised in that** the acetic acid content is maintained in the reaction medium of the first step at a value which is not less than 25 % by weight.

15. The process according to one of claims 1 to 14, **characterised in that** said halogenated promoter is selected from iodinated compounds, and precursors thereof.

16. The process according to claim 15, **characterised in that** said halogenated promoter is selected from the group consisting of iodine, methyl iodide, hydroiodic acid and acetyl iodide.

17. The process according to claim 16, **characterised in that** said halogenated promoter is methyl iodide.

18. The process according to one of claims 1 to 17, **characterised in that** the catalytic system further comprises a rhodium-based catalytic compound.

19. The process according to one of claims 1 to 18 **characterised in that** the overall concentration of the catalytic compound(s) is between 0.1 and 100 mmol/l in the reaction medium of the first step.

20. The process according to one of claims 1 to 19, **characterised in that** iodides in the form of an ionic compound which is soluble in said medium are maintained in the reaction medium of the first step.

21. The process according to one of claims 1 to 20, **characterised in that**, in the reaction step, the temperature is maintained between 150 and 250°C, preferably between 175° and 210°C, and the pressure at a value of between 0 and $200.10^5$ absolute Pa, preferably of less than $50.10^5$ absolute Pa.

22. The process according to one of claims 1 to 21, **characterised in that**, in the step of partial vaporisation, the temperature is maintained between 80 and 200°C and the pressure between 0 and $20.10^5$ absolute Pa.